# EUROPÄISCHE PATENTSCHRIFT

(11) **EP 1 663 938 B1**
(45) Veröffentlichungstag und Bekanntmachung des Hinweises auf die Patenterteilung: **14.10.2009**
(21) Anmeldenummer: 04763264.1
(22) Anmeldetag: 16.07.2004
(51) Int. Cl.: C07C 68/00

(54) **VERFAHREN ZUR HERSTELLUNG VON ORGANISCHEN CARBONATEN**
METHOD FOR THE PRODUCTION OF ORGANIC CARBONATES
PROCEDE DE PRODUCTION DE CARBONATES ORGANIQUES

(30) Priorität: 11.09.2003 DE 10341953
(43) Veröffentlichungstag der Anmeldung: 07.06.2006
(73) Patentinhaber: Lurgi GmbH, 60439 Frankfurt am Main (DE)
(72) Erfinder: BUCHOLD, Henning, 63452 Hanau (DE); EBERHARDT, Jürgen, 63110 Rodgau (DE); WAGNER, Ulrich, 06408 Biendorf (DE); WÖLK, Hans-Jörg, 83022 Rosenheim (DE)
(74) Vertreter: Meyer-Dulheuer, Karl-Hermann
(86) Internationale Anmeldenummer: PCT/EP2004/007911
(87) Internationale Veröffentlichungsnummer: WO 2005/028414

(56) Entgegenhaltungen:
- EP-A- 0 041 622
- EP-A- 0 478 073
- EP-A- 0 638 541
- EP-A- 0 866 051

## Beschreibung

Gegenstand der Erfindung ist ein zweistufiges Verfahren zur Herstellung organischer Carbonate.

Dimethylcarbonat und Diphenylcarbonat sind Zwischenprodukte der chemischen Industrie, die in einer Vielzahl von Anwendungsbereichen eingesetzt werden. So ist Dimethylcarbonat ein Ausgangsmaterial für aromatische Polycarbonate. Dimethylcarbonat wird mit Phenol zum Diphenylcarbonat umgeestert und in einer Schmelzpolymerisation mit Bisphenol zum aromatischen Polycarbonat umgesetzt (Daniele Delledonne; Franco Rivetti; Ugo Romano: "Developments in the production and application of dimethylcarbonate" Applied Catalysis A: General 221 (2001) 241 - 251). Dimethylcarbonat kann zur Verbesserung der Oktanzahl von Benzin eingesetzt werden und umweltproblematische Zuschlagstoffe wie MTBE ersetzen (Michael A. Pacheco; Christopher L. Marshall: "Review of Dimethyl Carbonate (DMC) Manufacture and it's Characteristics as a Fuel Additive" Energy & Fuels 11 (1997) 2 - 29). Dabei ist vor allem die leichte biologische Abbaubarkeit, die Ungiftigkeit und die gute Anwendbarkeit als Zuschlagstoff in Benzin zu nennen. Dimethylcarbonat hat eine Reihe von Anwendungen in der chemischen Synthese. Bei Temperaturen bei oder unter der Siedetemperatur von 90°C kann Dimethylcarbonat als Methoxylierungsmittel verwendet werden. Bei höheren Temperaturen um 160°C lässt sich Dimethylcarbonat als Methylierungsmittel einsetzen (Pietro Tundi; Maurizio Selva: "The Chemistry of Dimethyl Carbonate" Acc. Chem. Res. 35 (2002) 706 - 716).

Die bis ca. 1980 gebräuchliche Methode zur Herstellung von Dimethylcarbonat war die Alkoholyse von Phosgen mit Methanol (US 2,379,740, Pittsburgh Plate Glass Company 1941) oder (Kirk-Othmer, Encyclopedia of Chemical Technology, 3rd Edition, Volume 4, 758). Die Toxizität des Phosgens und das Entstehen von korrosivem Chlorwasserstoff stehen einer umweltbewussten kommerziellen Verwendung im großen Maßstab allerdings entgegen.

Der zur Zeit hauptsächlich eingesetzte Prozess ist die Umsetzung von Methanol mit Kohlenmonoxid und Sauerstoff an einem Kupferchloridkontakt, beschrieben in US 5,210,269 von Enichem (1993). Diese oxidative Carbonylierung verläuft über Kupfermethoxychlorid und eine anschließende Reaktion mit Kohlenmonoxid zu Dimethylcarbonat. Das Hauptproblem dieses Prozesses ist die Deaktivierung des Katalysators durch Wasser. Der desaktivierte Katalysator muss aufwendig regeneriert oder der Wassergehalt im Reaktor klein gehalten werden.

Eine Variante der oxidativen Carbonylierung ist eine zweistufige Reaktion über Methylnitrit. In einem Vorreaktor wird Methylnitrit aus Methanol, Stickstoffmonoxid und Sauerstoff synthetisiert, wobei Wasser als Nebenprodukt entsteht. Nach Entfernung des Wassers wird gasförmiges Methylnitrit in einem Festbettreaktor an einem Palladiumchloridkatalysator mit CO zu Dimethylcarbonat umgesetzt; das entstehende NO wird im Kreis geführt. Dieses Verfahren hat den Nachteil, dass der Umgang mit korrosivem Stickstoffmonoxid gefährlich ist.

Eine weitere Möglichkeit zur Herstellung von Dimethylcarbonat ist die Umesterung eines zyklischen Carbonats mit Methanol. Verfahren mit Ethylen- oder Propylencarbonat als Ausgangsmaterial sind bekannt (US 4,734,518 Texaco 1988; US 4,691,041 Texaco 1987). Ausgehend vom zyklischen Carbonat kann durch eine Umesterung mit Methanol das Dimethylcarbonat und gleichzeitig jeweils ein Mol des entsprechenden Diols synthetisiert werden. Die Alkylencarbonate lassen sich einfach darstellen. Der Nachteil dieser Methode ist die Coproduktion von Diolen bei der Herstellung des Dimethylcarbonats.

Die direkte Alkoholyse von Harnstoff mit Methanol ist eine weitere Möglichkeit zur Herstellung von Dimethylcarbonat. Die Synthese verläuft in zwei Schritten über den Carbamatsäuremethylester zum Dimethylcarbonat. Die Reaktionsgeschwindigkeit wird durch das gebildete Ammoniak stark gehemmt. Zur verbesserten Synthese wurden daher chemische und physikalische Methoden vorgeschlagen, um das entstehende Ammoniak zu entfernen.

Auch eine Fällung des entstehenden Ammoniaks durch BF₃ wurde erfolgreich durchgeführt (US 2,834,799; 1958), ist jedoch angesichts der entstehenden Salzfrachten unwirtschaftlich.

Das Entfernen des Ammoniaks (US 4,436,668; BASF1984) durch Zugabe von inertem Gas in einer zweiten Stufe lieferte bislang nur ungenügende Umsätze und Selektivitäten. Zur Verbesserung des Prozesses wurde eine zweite Stufe mit einem Katalysator-Reaktanden Dialkyl-isozyanat-alkoxyzinn (US 5,565,603; Exxon 1996; US 5,561,094; Exxon 1996) eingesetzt, der durch Methanol in situ hergestellt wird. Als Nachteil ist die Bereitstellung und Aufarbeitung des Katalysator-Reaktanden zu nennen.

Eine Alternative zur direkten Synthese ist der Einsatz eines zyklischen Carbonats (US 5,489,702 Mitsubishi Gas Chemical 1996; US 5,349,077; Mitsubishi Gas Chemical 1994). Hier wird in einem ersten Schritt ein Diol mit Harnstoff umgesetzt und ein zyklisches Alkylencarbonat mit 5 bzw. 6 Ringatomen synthetisiert. Im zweiten Prozessschritt wird das Alkylencarbonat mit Methanol umgeestert. Das Diol kann anschließend im Kreislauf gefahren werden.

Die bei der Alkoholyse hergestellten Zwischenprodukte müssen anschließend mit Methanol umgeestert werden, um das Dimethylcarbonat als Produkt zu erhalten. Die Umesterung ist eine katalysierte Reaktion. Als heterogene Katalysatoren werden basische Alkali- und Erdalkalimetalle bzw. Oxide eingesetzt. Beispiele für Alkali- oder Erdalkalimetalle in Zeolithen sind in US 6,365,767 der Exxon, Beispiele für Metalloxide sind in US 6,207,850 Mobil Oil genannt. Verfahren zur Umesterung von Ethylen- und Propylencarbonaten mit Alkoholen in Gegenstrom-Festbett-Rohrreaktoren mit homogenen oder heterogenen Katalystatoren (US 5,231,212; Bayer 1993; US 5,359,118; Bayer 1994) sowie ein Verfahrenspatent zur Synthese über Epoxide mit anschließender Umesterung an bifunktionellen Katalysatoren (US 5,218,135; Bayer 1993) sind ebenfalls bereits bekannt. Die Umesterung von zyklischen Carbonaten mit Alkoholen in einer Reaktivdestillation ist beschrieben (US 6,346,638; Asahi Kasei Kabushiki Kaisha 2002). Eine Reaktivextraktion mit Kohlenwasserstoffen oder Benzin als Phase zur Aufnahme des Dimethylcarbonats und einer polaren Phase aus Alkylencarbonat zur Aufnahme der Alkohole ist aus der US 5,489,703 bekannt.

Nur wenige dieser prinzipiell möglichen Synthesewege sind für eine technische und wirtschaftliche Realisierung aussichtsreich. Bei geforderten großen Mengen an Dimethylcarbonat kommen nur solche Verfahren in Betracht, die auch die notwendigen Rohstoffe in genügender Menge preiswert zur Verfügung haben. In den letzten Jahren wurde deshalb verstärkt daran gearbeitet, die Herstellung von organischen Carbonaten, vorzugsweise Dimethylcarbonat, basierend auf Harnstoff und Methanol technisch durchzuführen. Trotz zahlreicher Entwicklungen besitzen die bisher beschriebenen Verfahren zum Teil erhebliche Nachteile, so dass eine elegante technische Route zur Gewinnung von organischen Carbonaten wie DMC noch aussteht.

Als nachteilig erweist sich bei den bisher beschriebenen Verfahren:
- Die Reaktion von Harnstoff mit Methanol verläuft über die Zwischenstufe des Carbamates.
- Während der Reaktion wird Ammoniak abgespalten, welches entfernt werden muss.
- Die Reaktion verläuft wegen der unzureichenden Ammoniakabtrennung nur zu geringen Umsatzgraden.
- Ammoniak kann prinzipiell durch verschiedene Methoden aus dem Reaktionsgemisch entfernt werden, jedoch wird bei den aus dem Stand der Technik bekannten Verfahren hierbei ein zu entsorgender Feststoff gebildet oder ein großer Teil des eingesetzten Methanols mit ausgetragen.
- Große Mengen von Methanol müssen im Kreislauf gefahren werden.
- Ein für DMC entwickeltes Verfahren lässt sich nicht ohne Weiteres auf die Synthese anderer Carbonate ausweiten.

Von Vorteil wäre ein Verfahren, das die Synthese von Dimethylcarbonat und anderen organischen Carbonaten zulässt, ohne dass die oben genannten Nachteile vorhanden sind. Gleichzeitig wäre es wünschenswert, wenn durch ein neues Verfahren die für die Dimethylcarbonatsynthese notwendigen Zwischenprodukte einen möglichst hohen Siedepunkt hätten, so dass sie bei der notwendigen Entfernung des Ammoniaks nicht mit entfernt werden. Von besonderem Vorteil wäre, wenn ein Verfahren zur Verfügung stehen würde, welches nicht reine Stoffe als Hilfsstoffe für die Zwischenproduktherstellung verlangen würde, sondern Mischungen zulassen könnte, um einerseits eine verbesserte Löslichkeit für den Harnstoff zu erreichen und andererseits das Siedeverhalten positiv zu beeinflussen. Weiter wäre von Vorteil, wenn die hierzu eingesetzten Hilfsstoffe umweltneutral wären.

Mit dem hier vorgestellten neuen Verfahren zur Herstellung von organischen Carbonaten können diese Ziele erreicht werden.

Gegenstand der Erfindung ist deshalb ein Verfahren zur Herstellung von organischen Carbonaten, bei dem Harnstoff, ein substituierter Harnstoff, ein Salz oder Ester der Carbamidsäure oder eines ihrer N-substituierten Derivate (Alkyl-, Aryl-Reste wie Methyl-, Ethyl-, Phenyl-, Benzyl-)
- in einer ersten Stufe mit polymeren mehrfunktionalen Alkoholen wie Polyalkylenglykolen, Polyester-Polyolen oder Polyether-Polyolen der allgemeinen Formel I 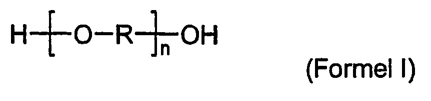 in dem R eine geradkettige oder verzweigte Alkylengruppe mit 2 bis12 Kohlenstoffatomen und n eine Zahl zwischen 2 und 20 bedeuten,
   oder vollständig oder teilweise hydrolysierten Polyvinylalkoholen der allgemeinen Formel II 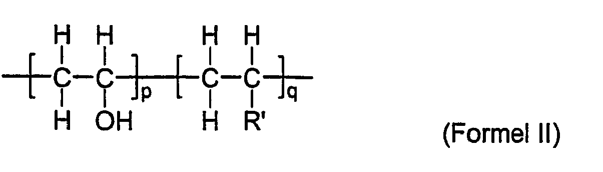 in der R' eine Alkyl-, Aryl- oder Acylgruppe mit 1 -12 Kohlenstoffatomen, p und q Zahlen zwischen 1-20 bedeuten
   oder in Mischungen dieser Verbindungen gelöst, ohne oder in Gegenwart eines die Ammoniakabspaltung begünstigenden Katalysator zu einer Carbonate und Carbamate enthaltenden Mischung umgesetzt, das dabei frei werdende Ammoniak oder das Amin aus der Reaktionsmischung durch ein Strippgas und/oder Dampf und/oder Vakuum entfernt wird und
- in einer zweiten Stufe (Umesterung) die Carbonate und Carbamate der polymeren Alkohole enthaltende Mischung mit einem Alkohol oder einem Phenol unter Bildung von deren Carbonaten und Rückbildung der polymeren Polyalkohole der Formeln I oder II umgesetzt wird.

Bisher werden für die Herstellung des Zwischenproduktes Carbamat nach dem Stand der Technik monomeres Glykol und monomere Diole mit Harnstoff eingesetzt (Michael A. Pacheco; Christopher L. Marshall: "Review of Dimethyl Carbonate (DMC) Manufacture and it's Characteristics as a Fuel Additive" Energy & Fuels 11 (1997) 2 - 29). Dies erfolgt in der ersten Stufe, um daraus die Carbonate dieser Alkohole zu erzeugen.

Überraschenderweise hat sich nun gezeigt, dass der Einsatz von polymeren Alkoholen (Polyolen) eine Reihe von wesentlichen Vorteilen gegenüber dem Stand der Technik aufweist.
• Polymere Alkohole und die daraus gebildeten Carbonate und Carbamate besitzen einen wesentlich höheren Siedepunkt als die bisher im Stand der Technik beschriebenen Monoalkohole, Diole und die daraus gebildeten Carbonate und Carbamate. Dies führt dazu, dass bei der Entfernung des bei der Reaktion entstehenden Ammoniaks durch Strippen oder Vakuum ein fast vollständiger Umsatz bei gleichzeitig minimalen Verlusten an diesen höher siedenden Alkoholen und Carbonaten bzw. Carbamaten erreicht wird. Dies ist bei Einsatz der nach dem Stand der Technik bekannten Methoden nicht möglich, da beim Strippen auch hohe Anteile der dort verwendeten Alkohole und des Glykolcarbonates bzw. Diolcarbonates mit aus dem Reaktionsgemisch ausgetrieben werden.
• Polymere Alkohole besitzen auf Grund ihres wasserähnlichen polaren Aufbaus eine höhere Löslichkeit für Harnstoff, substituierte Harnstoffe, Salze und Ester der Carbamidsäure und ihre N-substituierten Derivate als die bisher verwendeten langkettigen Monoalkohole bzw. Diole, so dass die Umsetzungen in homogener Lösung durchgeführt werden können. Durch die Veränderung der Kettenlänge n und der Größe der Nebengruppen R lassen sich die Löslichkeit und gleichzeitig der Siedepunkt der Mischung gezielt einstellen. Außerdem sind polymere Alkohole bei Temperaturen, bei denen vergleichbar lange Monoalkohole bzw. Diole bereits fest sind, noch flüssig.
• Darüber hinaus haben diese Hilfsstoffe eine einstellbare Viskosität, sind wenig korrosiv und eignen sich somit besonders für eine Kreislauffahrweise. Ferner sind sie nicht toxisch und damit umweltneutral
• Polymere Alkohole besitzen eine deutlich höhere chemische, thermische und mechanische Stabilität als die bisher verwendeten Stoffe, was für eine Rückführung (Kreislauffahrweise) der dieser Alkohole nach der Rückbildung in der zweiten Stufe von großem Vorteil ist, da die Verluste an polymeren Alkoholen aufgrund von Zersetzungs- bzw. thermischen Crackprozessen minimal sind.
• Normalerweise würde man nicht daran denken, polymere Alkohole für den Zweck der Zwischenproduktbildung einzusetzen, denn polymere Alkohole sind Mehrkomponentenmischungen, die sich verfahrenstechnisch schwieriger handhaben lassen als reine Hilfsstoffe. Die bei Einsatz von polymeren Alkoholen anfallende Mehrkomponentenmischung bereitet in der Regel bei der Weiterverarbeitung Schwierigkeiten. Gerade aber durch den Einsatz von polymeren Alkoholen kann hier ein verfahrenstechnischer Vorteil erzielt werden. Denn es hat sich überraschend gezeigt, dass es gar nicht notwendig ist, die anfallende Mehrkomponentenmischung aufzuarbeiten, sondern dass man sie direkt der zweiten Stufe (Umesterung) zuführen kann, ohne dass hierdurch Nachteile entstehen. Denn in der Umesterung mit niederen Alkoholen oder Phenolen bilden sich alle anfänglich vorhandenen polymeren Alkohole genau wie die üblicherweise verwendeten Monoalkohole bzw. Diole vollständig zurück. Diese können dann wieder der ersten Stufe zugeführt werden (Kreislauffahrweise).

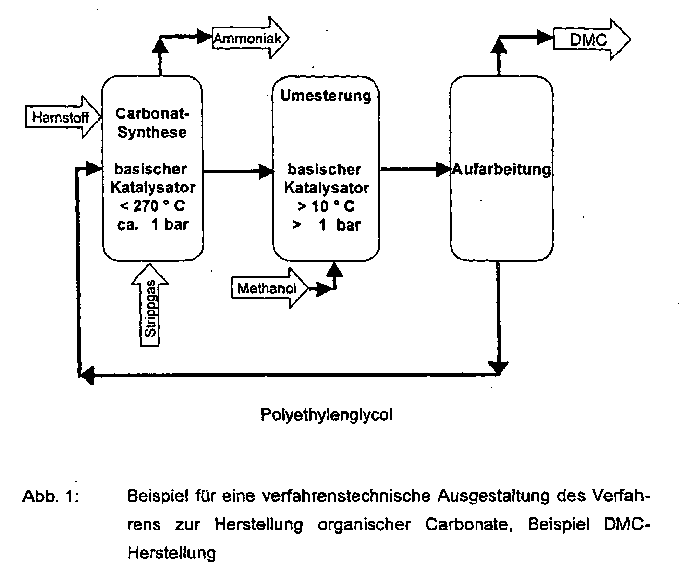

Eine vorteilhafte Ausgestaltung eines Verfahrens zur Herstellung von organischen Carbonaten ist in Abbildung 1 dargestellt.

Wichtige Merkmale des erfindungsgemäßen Verfahrens sind:
- Umsetzung von Harnstoff, substituierten Harnstoffen, Salzen und Estern der Carbamidsäure und ihre N-substituierten Derivate mit polymeren Alkoholen zu hochsiedendenden Carbonaten in einer Stufe.
- Hohe Umsätze und Ausbeuten durch simultanes Entfernen (Strippen mit Gas und/oder Dampf bzw Anlegen von Vakuum) des bei der Reaktion entstehenden Ammoniaks bei minimalen Verlusten von Alkoholen Carbonaten und Carbamaten.
- Die Reaktion in der ersten Stufe erfordert keinen Katalysator. Jedoch kann durch den Einsatz basischer Katalysatoren eine weitere Erhöhung der Reaktionsgeschwindigkeit erreicht werden.
- Erzeugung der gewünschten Carbonate in einer zweiten Stufe durch einfache Umesterung der hochsiedenden Carbonate aus einer ersten Stufe.
- Die als Hilfsstoffe eingesetzten polymeren Alkohole werden im Kreislauf geführt, ohne dass es einer aufwendigen Aufarbeitung bedarf.
- Es handelt sich um ein variables Verfahren, das auch zur Herstellung von Carbonaten von geradkettigen oder verzweigten aliphatischen Alkoholen mit 2 - 10 Kohlenstoffatomen, nicht nur für Dimethylcarbonat, eingesetzt werden kann.
- Ebenso kann das Verfahren auch zur Herstellung von Carbonaten von substituierten Phenolen verwendet werden, die 1 - 4 Kohlenstoffatome aufweisende Alkylgruppen tragen, also nicht nur für die Herstellung von Diphenylcarbonat, weil die Siedepunkte der Zwischenprodukte hoch und weit entfernt von den Siedepunkten der in der Umesterung eingesetzten Alkohole oder Phenole sind. Dadurch ist eine bessere Trennung von Zielcarbonat und zurückgebildeten Hilfsstoffen über einen weiteren Temperaturbereich möglich.

Die Wirksamkeit des hier vorgestellten neuen Verfahrens zur Herstellung von organischen Carbonaten soll anhand einiger Beispiele verdeutlicht werden.

Das erfindungsgemäße Verfahren wird in beiden Stufen in vorteilhafter Weise bei Temperaturen zwischen 10°C und 270°C durchgeführt. In der ersten Stufe wird unter Normaldruck und Zudosierung eines zum Austreiben des gebildeten Ammoniaks geeigneten Gases oder Dampfes in Gegenwart von Katalysatoren gearbeitet. Hierfür sind alkalisch reagierende Salze, Oxide, Hydroxide, Alkoholate der ersten und zweiten Hauptgruppe oder der 1. Bis 8. Nebengruppe des Periodensystems, basische Zeolithe oder polymere Ionenaustauscher als Katalysatoren geeignet. Beispielsweise sind Magnesium- oder Zinkkatalysatoren, die sowohl als Oxid oder auch als Acetat eingesetzt werden können katalytisch wirksam. Eine wichtiger Einflußgröße ist die Entfernung des Ammoniaks durch Strippen mit Gas, Dampf oder Vakuum.

Die zweite Stufe beinhaltet die Umesterung des Carbonats oder Carbamates mit einem Alkohol oder einem Phenol und kann in Anwesenheit der gleichen basischen Katalysatoren wie die erste Stufe durchgeführt werden. Als besonders vorteilhaft hat sich die Verwendung von quaternären Ammoniumsalzen wie Tetrabutylammoniumacetat oder Tetrabutylammoniumbromid oder Magnesiumalkoholaten erwiesen. Die Umesterung ist eine Gleichgewichtsreaktion, deren Lage eher auf der Seite der Ausgangsstoffe liegt, so dass eine simultane Reaktion und Stofftrennung sinnvoll ist. Temperaturen zwischen 10°C und 270°C erlauben eine relativ zügige Umsetzung in dieser Stufe.

Die Erfindung wird durch die nachstehenden Versuche im einzelnen erläutert.

### Versuchsaufbau und Durchführung

Alle Versuche zur Umsetzung des in einem polymeren Alkohol gelösten Harnstoffes wurden in einem 150 ml Glasdoppelmantelreaktor mit Heizmantel, Begasungseinrichtung und Rückflusskühler durchgeführt. Ein Tropfenabscheider vor dem Eintritt in den Rückflusskühler verhinderte den Austrag von mitgerissener Flüssigkeit. Als Strippgas wurde Stickstoff eingesetzt. Vakuum konnte über eine angeschlossene Membranpumpe eingesetzt werden. Proben wurden diskontinuierlich gezogen.

### Untersuchungen mit Polyethylenglykol

Polyethylenglykol ist als Reaktand geeignet, da er eine Reihe interessanter Eigenschaften aufweist. Die Umsetzung dieses zweiwertigen Alkohols mit Hamstoff kann prinzipiell zwei Produkte erzeugen. Diese beiden langkettigen Carbonate sind:

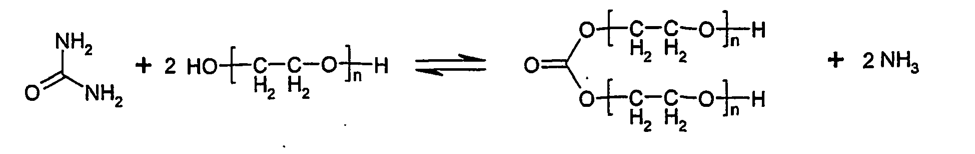

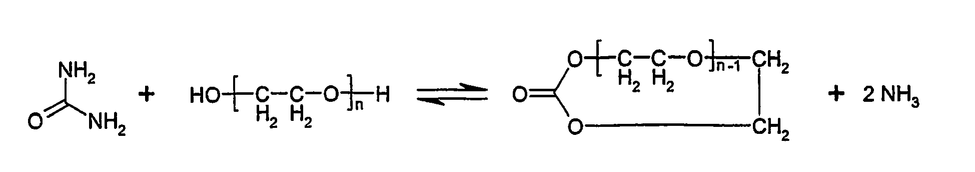

Beide Carbonate eignen sich für die Umesterung in der zweiten Stufe mit Methanol zum gewünschten Produkt. Die Untersuchungen zeigten, dass die Reaktion zum zyklischen Carbonat wahrscheinlicher ist, da die Reaktion in einem Verhältnis von 1 Mol Harnstoff zu 1 Mol Polyethylenglykol abläuft. In beiden Fällen ist das Carbamat als Zwischenprodukt zu beobachten:

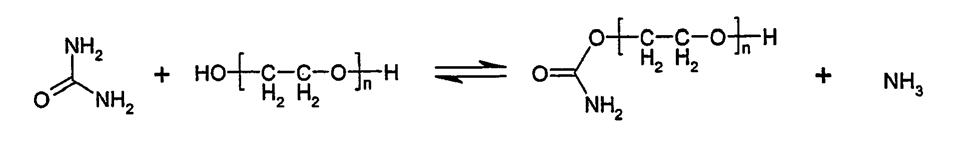

### Verwendung unterschiedlicher Katalysatoren

Die in der Patentliteratur genannten Katalysatoren umfassen eine Reihe von Metalloxiden. In den erfindungsgemäß durchgeführten Versuchen kamen pulverförmige Oxide und Acetate zum Einsatz. Diese wurden in Massenverhältnissen zwischen 5 und 25 Gewichtsprozent eingesetzt. Titandioxid, Zinkoxid, Magnesiumoxid und Magnesiumacetat wurden als mögliche Katalysatoren untersucht.

Dabei zeigten sich nur geringe Unterschiede in den Reaktionsverläufen für diese verschiedenen Katalysatoren. Die Reaktionsgeschwindigkeit war auch bei 150°C sehr gering und selbst nach 16 Stunden kein Ende der Umsetzung absehbar. Die Beschleunigung der Reaktion war bei Magnesiumacetat, Magnesiumoxid und Zinkoxid nahezu gleich groß. Diese Verbindungen zeigten eine deutlich bessere katalytische Aktivität als Titanverbindungen.

Eine Erhöhung der Katalysatormenge wurde untersucht, brachte aber nicht den erhofften Unterschied in der Reaktionsgeschwindigkeit. Bei 150°C war so gut wie kein Unterschied zwischen den Versuchen mit 6 bzw. 20 g Magnesiumacetat zu erkennen. Auch bei höherer Temperatur von 200°C war nach einer anfänglich schnelleren Produktentwicklung kein gravierender Unterschied in der erhaltenen Produktmenge zu erkennen.

### Variation der Temperatur

Vorversuche an Reaktionen von Harnstoff mit Polyethylenglykol haben gezeigt, dass unterhalb von etwa 140°C so gut wie keine Reaktion zu beobachten ist. Als minimale Versuchstemperatur wurden daher 150°C gewählt. Bei den Versuchen mit Titandioxid wurde ein eher moderater Stickstoffvolumenstrom zum Austreiben des Ammoniaks verwendet. Ein deutlicher Einfluss der Reaktionstemperatur beim Anheben des Levels von 150 auf 200°C ist im Verlauf des zeitlichen Konzentrationsverlaufes des Polyethylenglykols nicht zu erkennen. Es zeigte sich, dass bei 200°C nach ca. 5 Stunden ein nahezu vollständiger Umsatz erreicht wurde, während bei 150°C sehr wenig Produkt entstanden ist.

### Einsatz von Vakuum oder Strippgas (Stickstoff)

Als Haupteinflussparameter für die Umsetzung von Harnstoff mit Polyethylenglykol wurde das Austreiben des gebildeten Ammoniaks aus der Reaktionsmischung durch Vakuum oder Strippen mit Stickstoff identifiziert. Das Arbeiten unter Vakuum wurde mit zwei Versuchen bei einem Druck von 300 mbar untersucht. Es konnte eine merkliche Verbesserung des Umsatzverhaltens im Vergleich zur Reaktion ohne Austreiben des gebildeten Ammoniaks bei Umgebungsdruck festgestellt werden. Noch bessere Ergebnisse ergaben sich bei der Begasung der Reaktionsmischung mit Stickstoff. Eine Variation des Volumenstroms zeigte einen deutlichen Einfluss auf die Umsetzung von Harnstoff mit Polyethylenglykol.

Die anschließende Umesterung der polymeren Carbonate und Carbamate wurde mit Methanol bzw. Phenol in Gegenwart von Tetraalkylammoniumsalzen bzw. Magnesiummethylat durchgeführt, wobei nach Aufarbeitung der Reaktionsprodukte die gewünschten Carbonate erhalten werden.

Durch das erfindungsgemäße Verfahren ist die Gewinnung von organischen Carbonaten über die Zwischenstufe einer Mischung organischer Carbonate und Carbamate mit polymeren Resten durch die Reaktion von Harnstoff oder Harnstoffderivaten mit polymeren Alkoholen wie Polyethylenglykol bei Normaldruck, Temperaturen von bis zu 270°C und Zugabe von bis zu 5 Gewichtsprozent von alkalisch reagierenden Katalysatoren möglich. Die in der ersten Stufe erhaltenen Produkte sind ausschließlich organische Carbonate und Carbamate der entsprechenden polymeren Alkohole. Bei Reaktionszeiten von etwa 5 Stunden wurden Umsätze von über 90% erzielt.

Eine wichtige Einflussgröße für die Erzielung hoher Umsätze ist der Volumenstrom des Strippgases. Bei ausreichend hohen Volumenströmen ist die Entfernung des Ammoniaks nicht mehr der geschwindigkeitsbestimmende Schritt.

Die Umsetzung des in der ersten Stufe erzeugten Carbonats oder Carbamats mit Methanol verläuft mit einem basischen Katalysator bei Anwendung eines leicht erhöhten Drucks von ca. 6 bar bei einer Temperatur von ca. 140°C relativ schnell ab. Das Gleichgewicht ist nach weniger als 1 Stunde im Batchbetrieb eingestellt. Als Katalysator wurde ein quaternäres Ammoniumsalz eingesetzt, welches gute katalytischen Eigenschaften zeigte. Noch höhere Reaktionsgeschwindigkeiten wurden durch Verwendung von Magnesiummethylat erreicht.

Bei der erfindungsgemäßen Koppelung beider Stufen wird der als Hilfsalkohol verwendete polymere Alkohol nach Abtrennung des Dimethylcarbonats oder des Diphenylcarbonats in die erste Stufe wieder zurückgeführt. Durch die Führung des Verfahrens im Kreislauf werden Verluste des polymeren Hilfsalkohols vermieden, so dass das Verfahren als außerordentlich wirtschaftlich anzusehen ist.

## Patentansprüche

1. Verfahren zur Herstellung von organischen Carbonaten, **dadurch gekennzeichnet, dass** Harnstoff, ein substituierter Harnstoff, ein Salz oder Ester der Carbamidsäure oder eines ihrer N-substituierten Derivate
- in einer ersten Stufe mit polymeren mehrfunktionalen Alkoholen wie Polyalkylenglykole, Polyester-Polyolen oder Polyether-Polyolen der allgemeinen Formel I 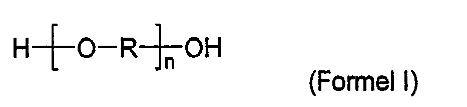 in dem R eine geradkettige oder verzweigte Alkylengruppe mit 2 bis 12 Kohlenstoffatomen und n eine Zahl zwischen 2 und 20 bedeuten, oder
- mit vollständig oder teilweise hydrolysierten Polyvinylalkoholen der allgemeinen Formel II 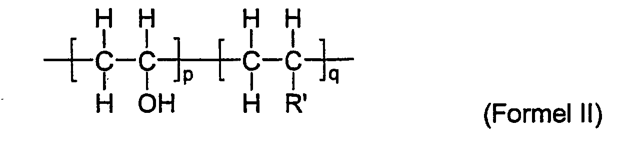 in der R' eine Alkyl-, Aryl- oder Acylgruppe mit 1 -12 Kohlenstoffatomen, p und q Zahlen zwischen 1 - 20 bedeuten,
- oder in Mischungen dieser Verbindungen gelöst, ohne oder in Gegenwart eines die Ammoniakabspaltung begünstigenden Katalysators zu einer Carbonate und Carbamate enthaltenden Mischung umgesetzt,
und gleichzeitig das dabei frei werdende Ammoniak oder das Amin aus der Reaktionsmischung durch ein Strippgas und/oder Dampf und/oder Vakuum entfernt wird,
und in einer zweiten Stufe (Umesterung) eine die Carbonate und Carbamate der polymeren Alkohole enthaltende Mischung mit einem Alkohol oder einem Phenol unter Bildung von deren Carbonaten und Rückbildung der polymeren Polyalkohole der Formeln I oder II umgesetzt werden.

2. Verfahren nach Anspruch 1, **dadurch gekennzeichnet, dass** die in der zweiten Stufe zurückgebildeten polymeren Polyalkohole der Formeln I oder II vollständig oder teilweise wieder der ersten Stufe zugeführt werden.

3. Verfahren nach den Ansprüchen 1 und 2, **dadurch gekennzeichnet, dass** beide Stufen bei Temperaturen zwischen 10° und 270°C durchgeführt werden.

4. Verfahren nach den Ansprüchen 1 bis 3, **dadurch gekennzeichnet, dass** in beiden Stufen alkalisch reagierende Salze, Oxide, Hydroxide, Alkoholate mit Elementen der Gruppen Ia, Ib, IIa, IIb, IIIa, IIIb, IVa, IVb, Va, Vb, VIb, VIIb, VIIIb, des Periodensystems, basische Zeolithe, polymere Ionenaustauscher oder Tetraalkylammoniumsalze oder Triphenylphosphine oder tertiäre Amine als Katalysatoren eingesetzt werden.

5. Verfahren zur Herstellung von Dimethylcarbonat und/oder anderer organischer Carbonate nach den Ansprüchen 1 bis 4, **dadurch gekennzeichnet, dass** in der zweiten Stufe Methylalkohol und/oder geradkettige oder verzweigte aliphatische Alkohole mit 2 bis 10 Kohlenstoffatomen und/oder zyklische Alkohole mit 5 bis 10 Kohlenstoffatomen oder Phenol und/oder substituierte Phenole verwendet werden, die 1 bis 4 Kohlenstoffatome aufweisende Alkylgruppen tragen und/oder aromatische Alkohole, die 6 bis 20 Kohlenstoffatome aufweisen und /oder Heteroatome enthaltende Alkohole und/oder eine Mischung dieser Stoffe verwendet werden.

## Claims

1. A method for producing organic carbonates, **characterized in that** urea, substituted urea, a salt or ester of carbamic acid or one of the N-substituted derivatives thereof
- is dissolved in a first stage with polymeric multifunctional alcohols such as polyalkylene glycols, polyester-polyols or polyether-polyols of general Formula I 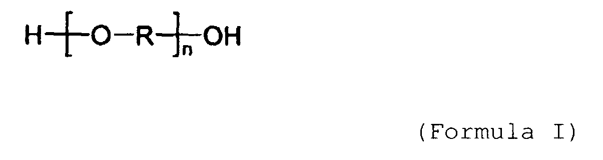
wherein R represents a straight-chain or branched alkylene group having 2 to 12 carbon atoms and n represents a number between 2 and 20; or
- with fully or partially hydrolyzed polyvinyl alcohols of general Formula II 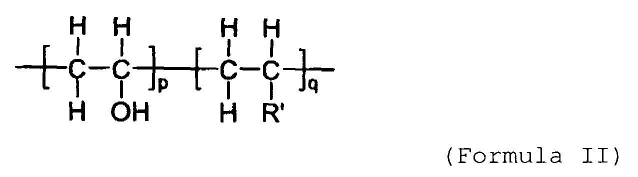
wherein R' represents an alkyl, aryl or acyl group having 1 - 12 carbon atoms, p and q represent numbers between 1 - 20;
- or in mixtures of these compounds; reacted without or in the presence of a catalyst promoting ammonia separation to a mixture comprising carbonates and carbamates;
- and the ammonia being liberated or the amine from the reaction mixture is simultaneously removed by stripping gas and/or steam and/or vacuum;
- and, in a second stage (transesterification), a mixture comprising the carbonates and carbamates of the polymeric alcohols is reacted with an alcohol or a phenol to form the carbonates thereof and re-form the polymeric polyalcohols of Formula I or Formula II.

2. A method according to claim 1, **characterized in that** said polymeric polyalcohols of Formula I or Formula II re-formed in the second stage are fully or partially returned to the first stage.

3. A method according to claims 1 and 2, **characterized in that** both stages are performed at temperatures between 10°C and 270°C.

4. A method according to claims 1 to 3, **characterized in that** in both stages alkaline-reacting salts, oxides, hydroxides, alkoxides with elements of groups Ia, Ib, IIa, IIb, IIIa, IIIb, IVa, IVb, Va, Vb, VIb, VIIb, VIIIb of the periodic table, basic zeolites, polymeric ion exchangers or tetraalkylammonium salts or triphenylphosphines or tertiary amines are used as catalysts.

5. A method for producing dimethyl carbonate and/or other organic carbonates according to claims 1 to 4, **characterized in that** in the second stage methyl alcohol and/or straight-chain or branched aliphatic alcohols having 2 to 10 carbon atoms and/or cyclic alcohols having 5 to 10 carbon atoms or phenol and/or substituted phenols, which comprise alkyl groups having 1 to 4 carbon atoms, are used and/or aromatic alcohols having 6 to 20 carbon atoms and/or alcohols containing heteroatoms and/or a mixture of these substances is used.

## Revendications

1. Procédé de préparation de carbonates organiques, **caractérisé en ce que** l'on dissout, dans une première étape, de l'urée, une urée substituée, un sel ou un ester de l'acide carbamique ou de l'un de ses dérivés N-substitués
- avec des alcools polyfonctionnels polymères comme les polyalkylène glycols, des polyols de polyester odes des polyols de polyéther répondant à la formule générale I 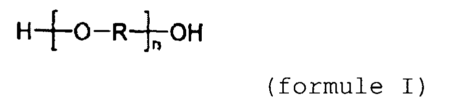
dans laquelle R représente un groupe alkylène renfermant 2 à 12 atomes de carbone, n étant un nombre compris entre 2 et 20, ou
- avec des alcools polyvinyliques complètement ou partiellement hydrolysés répondant à la formule générale II 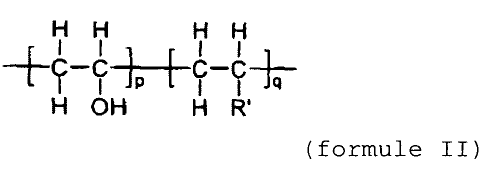
dans laquelle R' représente une groupe alkyle, aryle ou acyle renfermant 1 à 12 atomes de carbone, p et q étant des nombres compris entre 1 et 20,
- ou dans des mélanges desdits composés afin d'obtenir, par une réaction réalisée en présence ou en absence d'un catalyseur favorisant la libération d'ammoniaque, un mélange contenant des carbonates et des carbamates,
- l'ammoniaque dégagé ou l'amine issue du mélange réactionnel étant en même temps éliminé(e) par un gaz d'entraînement et/ou de la vapeur et/ou en appliquant un vide,
- pour faire réagir, dans une deuxième étape (transestérification), un mélange contenant les carbonates et les carbamates des alcools polymères avec un alcool ou un phénol, ce qui conduit à la formation de leurs carbonates et à la reconstitution des polyalcools polymères des formules I et II.

2. Procédé selon la revendication 1, **caractérisé en ce que** lesdits polyalcools polymères des formules I et II reconstitués dans la deuxième étape sont complètement ou partiellement recyclés vers la première étape.

3. Procédé selon les revendications 1 et 2, **caractérisé en ce que** les deux étapes sont réalisées à des températures comprises entre 10 ° et 270 °C.

4. Procédé selon les revendications 1 à 3, **caractérisé en ce que**, dans les deux étapes, on met en oeuvre des sels, des oxydes, des hydroxydes, lesdits composés présentent une réaction alcaline, avec des éléments des groupes Ia, Ib, IIa, IIb, IIIa, IIIb, IVa, IVb, Va, Vb, VIb, VIIb, VIIIb du tableau périodique, des zéolithes basiques, des échangeurs d'ions polymères ou des sels de tetraalkylammonium ou des triphénylphophines ou des amines tertiaires, lesdits composés servant de catalyseurs.

5. Procédé de préparation de diméthylcarbonate et/ou d'autres carbonates organiques selon les revendications 1 à 4, **caractérisé en ce que**, dans la deuxième étape, on utilise de l'alcool méthylique et/ou des alcools aliphatiques linaires ou ramifiés renfermant 2 à 10 atomes de carbone et/ou des alcools cycliques renfermant 5 à 10 atomes de carbone ou du phénol et/ou des phénols substitués portant des groupes alkyle comportant 1 à 4 atomes de carbone et/ou des alcools aromatiques comportant 6 à 20 atomes de carbone et/ou des alcools comportant des hétéroatomes et/ou qu'on utilise un mélanges de ces substances.
